# EUROPEAN PATENT SPECIFICATION

(11) **EP 2 722 059 B1**
(45) Date of publication and mention of the grant of the patent: **17.05.2017**
(21) Application number: 13189606.0
(22) Date of filing: 22.10.2013
(51) Int. Cl.: A61K 49/00, G01N 33/574

(54) **Photodynamic diagnostics of tissue fluids caused by oncological pathology**
Photodynamische Diagnose von Gewebeflüssigkeiten durch onkologische Pathologie
Diagnostics photodynamiques de fluides de tissus induits par une pathologie oncologique

(30) Priority: 22.10.2012 LT 2012098
(43) Date of publication of application: 23.04.2014
(73) Proprietor: I.I. Lazeriu ir Bangu Medicinos Moksliné Klinika, 08401 Vilnius (LT); Nacionalinis vézio institutas, 08660 Vilnius (LT)
(72) Inventor: Plesniene, Laima, LT-08401 Vilnius (LT); Plesnys, Albinas, LT-08401 Vilnius (LT); Vaisnoras, Rimantas, LT-08106 Vilnius (LT)
(74) Representative: Pakeniene, Ausra

(56) References cited:
- EP-A1- 1 203 944
- EP-A1- 1 797 903
- WO-A1-2011/161933
- WO-A1-2013/184830
- FLVIA RODRIGUES DE OLIVEIRA SILVA ET AL: "Enhancement of blood porphyrin emission intensity with aminolevulinic acid administration: A new concept for photodynamic diagnosis of early prostate cancer", PHOTODIAGNOSIS AND PHOTODYNAMIC THERAPY, vol. 8, no. 1, 17 November 2011 (2011-11-17), pages 7-13, XP028145245, ISSN: 1572-1000, DOI: 10.1016/J.PDPDT.2010.12.006 [retrieved on 2010-12-23]
- HIDEKI SAKURAI ET AL: "BASIC STUDY ON PHOTODYNAMIC THERAPY IN LIVER CELL CARCINOMA WITH REFERENCE TO EXPERIMENTAL LIVER TUMOR IN RABBITS AND DISTRIBUTION OF PHOTOSENSITIVE PH-1126", JOURNAL OF TOKYO MEDICAL COLLEGE, vol. 48, no. 6, 25 July 1990 (1990-07-25), pages 786-794, XP055103483,
- MALINI OLIVO ET AL: "New Frontier in Hypericin-Mediated Diagnosis of Cancer with Current Optical Technologies", ANNALS OF BIOMEDICAL ENGINEERING, KLUWER ACADEMIC PUBLISHERS-PLENUM PUBLISHERS, NE, vol. 40, no. 2, 29 November 2011 (2011-11-29), pages 460-473, XP035016175, ISSN: 1573-9686, DOI: 10.1007/S10439-011-0462-7
- PYTEL AKOS ET AL: "New aspect of photodynamic diagnosis of bladder tumors: Fluorescence cytology", UROLOGY, BELLE MEAD, NJ, US, vol. 59, no. 2, 31 January 2002 (2002-01-31), pages 216-219, XP009173544, ISSN: 0090-4295
- STEPHAN TAUBER ET AL: "Fluorescence cytology of the urinary bladder", UROLOGY, vol. 61, no. 5, 29 April 2003 (2003-04-29) , pages 1067-1071, XP055127025, ISSN: 0090-4295, DOI: 10.1016/S0090-4295(02)02554-2
- STEPHAN TAUBER ET AL: "Integral spectrophotometric analysis of 5-aminolaevulinic acid-induced fluorescence cytology of the urinary bladder", BJU INTERNATIONAL, vol. 97, no. 5, 6 April 2006 (2006-04-06), pages 992-996, XP055127026, ISSN: 1464-4096, DOI: 10.1111/j.1464-410X.2006.06094.x
- Muhammad W Saif ET AL: "Management of ascites due to gastrointestinal malignancy", Ann Saudi Med.2009 Sep-Oct; 29(5):, 1 March 2009 (2009-03-01), pages 369-377., XP055183892, Retrieved from the Internet: URL:http://www.ncbi.nlm.nih.gov/pmc/articl es/PMC3290049/?report=printable [retrieved on 2015-04-17]
- M. Fiegl: "Combination of Cytology, Fluorescence In Situ Hybridization for Aneuploidy, and Reverse-Transcriptase Polymerase Chain Reaction for Human Mammaglobin/Mammaglobin B Expression Improves Diagnosis of Malignant Effusions", Journal of Clinical Oncology, vol. 22, no. 3, 22 December 2003 (2003-12-22), pages 474-483, XP055183895, ISSN: 0732-183X, DOI: 10.1200/JCO.2004.06.063
- Ben Davidson: "Biological Characteristics of Cancers Involving the Serosal Cavities - Critical Reviews(TM) in Oncogenesis, Volume 13, 2007, Issue 3 - Begell House Digital Library", , 1 January 2007 (2007-01-01), XP055183906, Retrieved from the Internet: URL:http://www.dl.begellhouse.com/journals /439f422d0783386a,1f927aaa3b561c16,2cb443a 0154391b4.html [retrieved on 2015-04-17]
- Akihiko Kawahara ET AL: "Cytological features of cystadenocarcinoma in cyst fluid of the parotid gland: Diagnostic pitfalls and literature review", Diagnostic Cytopathology, 1 January 2009 (2009-01-01), pages NA-NA, XP055183908, ISSN: 8755-1039, DOI: 10.1002/dc.21232
- TER BORG E J ET AL: "Monoarthritis of the elbow due to metastatic colon carcinoma: diagnosis based on the presence of adenocarcinoma cells in synovial fluid", RHEUMATOLOGY INTERNATIONAL ; CLINICAL AND EXPERIMENTAL INVESTIGATIONS, SPRINGER, BERLIN, DE, vol. 28, no. 11, 26 April 2008 (2008-04-26), pages 1177-1178, XP019626043, ISSN: 1437-160X
- Anja C Roden ET AL: "Seroma-associated primary anaplastic large-cell lymphoma adjacent to breast implants: an indolent T-cell lymphoproliferative disorder", Modern Pathology, vol. 21, no. 4, 25 January 2008 (2008-01-25), pages 455-463, XP055183953, ISSN: 0893-3952, DOI: 10.1038/modpathol.3801024
- H. L. Gornik: "Abnormal Cytology Predicts Poor Prognosis in Cancer Patients With Pericardial Effusion", Journal of Clinical Oncology, vol. 23, no. 22, 6 June 2005 (2005-06-06), pages 5211-5216, XP055183957, ISSN: 0732-183X, DOI: 10.1200/JCO.2005.00.745

## Description

### Field of invention

The invention is related to biomedicine in particular to diagnostics of tissue fluids caused by an oncological pathology.

### Background of the invention

Accumulation of tissue fluids caused by malignant tumor in pleura and peritoneum is one of the most common problems for clinicians in their daily practice.

Neoplastic process may cause discharge of tissue fluids and subsequent accumulation in various cavities of a body - in most common cases in pleural and peritoneal cavities (less commonly in various neoplastic cysts, cavities of joints, etc.).

Ascites is a pathological accumulation of fluid within a peritoneal cavity. Ascites may be caused by [1]. Lombardi G, Zustovich F, Nicoletto MO, Donach M, Artioli G, Pastorelli D. Diagnosis and treatment of malignant pleural effusion: a systematic literature review and new approaches. Am J Clin Oncol 2010; 33 (4): 420-3.: various liver pathologies, mostly the liver cirrhosis (81 %), tumor (10 %), cardiac disease (3 %), tuberculosis (2 %), dialyses (1 %), pancreatic disease (1 %), peritoneal inflammatory pathology and other causes (2 %).

Tumor pathology accounts for 7 - 10 % of all ascites and 16.5 - 30 % of all pleurisy cases.

In most cases accumulation of fluid in pleura is caused by:
- Infection (pleurisy emerges when a subject is having pneumonia, tuberculosis);
- Impaired blood circulation and lymph circulation in lungs (various conditions causing stasis in lungs, thromboembolism in the lung arteries);
- Kidney diseases;
- Liver cirrhosis;
- Tumor damage to pleural layers (lung cancer, metastasis of a cancer of other organs, pleural mesothelioma);
- Damage to integrity of pleural layers caused by a trauma;
- Immune inflammation of pleural layers;
- Acute pancreatitis.

Normally, neoplastic process causes accumulation of exudates and only in rare cases transudates.

Causes for pleural transudate and exudate are different. Depending on the nature of pleural liquid, causes for accumulation of liquid in pleura [2] are as following:
- Most frequent causes of transudate (comprising 24 - 25 % of all cases of accumulation of pleural liquids):
   ∘ Cardiac failure - 14.5 %;
   ∘ Nephrotic syndrome - 5.5 %;
   ∘ Liver cirrhosis - 2.5 %l;
   ∘ Other pathology - 1.5 %.
- Most frequent causes of exudate (comprising 75 - 76 % of all cases of accumulation of pleural liquids):
   ∘ Neoplasia - 16.5%;
   ∘ Pneumonia - 13%;
   ∘ Empyema - 9%;
   ∘ Tuberculosis - 6%;
   ∘ Pulmonary embolism - 5.5%;
   ∘ Trauma - 3%;
   ∘ Hemothorax - 2%;
   ∘ Splenic abscess - 1.5%;
   ∘ Uraemia - 1%;
   ∘ Chylothorax - 1%;
   ∘ Pseudochylothorax - 0.5%;
   ∘ Pyopneumothorax - 0.5%;
   ∘ Unexplained - 16%.

According to different authors tumor pathology causes 16.5 - 30 % of all pleurisies, 42 - 77 % of all exudative pleurisies [3]. Marel M, Zrustova M, Stasny B, Light RW. The incidence of pleural effusion in a well-defined region: epidemiologic study in central Bohemia. Chest 1993; 104 (5): 1486-9; [4]. Valdes L, Alvarez D, Valle JM, Pose A, San Jose E. The etiology of pleural effusions in an area with high incidence of tuberculosis. Chest 1996; 109 (1): 158-62; and 40 % of all chronic pleurisies which in most cases are recurrent [5]. Valdes L, Alvarez D, Valle JM, Pose A, San Jose E. The etiology of pleural effusions in an area with high incidence of tuberculosis. Chest 1996; 109 (1): 158-62. 75% of all neoplastic pleurisies are caused by lung cancer, breast and ovaries cancer metastasis, mesothelioma and lymphoma. [6]. Johnston WW. The malignant pleural effusion. A review of cytopathological diagnoses of 584 specimens from 472 consecutive patients. Cancer 1985; 56 (4): 905-9.

Lung cancer causes one third of all neoplastic pleurisies [7]. Rodriguez-Panadero F, Romero-Romero B. Management of malignant pleural effusions. Curr Opin Pulm Med 2011; 17: 269-73. Autopsy of patients who had died from cancer revealed that 15 % of said patients had accumulation of tissue fluids in body cavities due to oncological pathologies [8]. Rodriguez-Panadero F, Borderas Naranjo F, Lopez-Mejias J. Pleural metastatic tumours and effusions. Frequency and pathogenic mechanisms in a post-mortem series. Eur Respir J 1989; 2 (4): 366-9. In the USA alone, every year more than 150,000 patients are diagnosed with tissue fluid accumulations due to oncological pathologies [9]. Chernow B, Sahn SA. Carcinomatous involvement of the pleura: an analysis of 96 patients. Am J Med 1977; 63: 695-702; [10]. Bille A, Borasio P, Gisabella M, Errico L, Gatherer R, Ardissone F. Suitable device for thoracoscopic talc poudrage in malignant pleural effusion. Gen Thorac Cardiovasc Surg 2011; 59 (7): 522-4; [11]. Cheong JC, Choi WH, Kim DJ, Park JH, Cho SJ, Choi CS, Kim JS. Prognostic significance of computed tomography defined ascites in advanced gastric cancer. J Korean Surg Soc 2012; 82: 219-26; [12]. Eskander RN, Tewari KS. Emerging treatment options for management of malignant ascites in patients with ovarian cancer. Int J Womens Health 2012; 4: 395-404. Even though in most cases pleurisy and ascites manifests in patients with a terminal stage cancer, pleural liquid as the first symptom of the cancer is diagnosed for as much as 41 % of all oncological patients with a malignant pleurisy [13]. Grannis Jr. FW, Lai L, Kim JY. Fluid complications. Cancer Management: 14th edition 2011.

Nevertheless as much as 50 % of all pleurisies that are diagnosed for oncological patients are non-malignant.

During cytological tests of pleural punctate, oncological pathology is diagnosed for 60 % of all subjects diagnosed with malignant pleurisies, the rest require pleural biopsy, whereby even with aid of a computer tomography, accurate diagnoses amounts only up to 87 % amongst all patients, sensitivity of the usually used Abrams method by a needle biopsy of a pleura amounts only up to 47 %.

During cytological tests of ascites, cancer cells are found only in 40 - 60 % of all cases of ascites, caused by oncological pathologies. Other tests' indicators, characteristic to malignant ascites, such as high level of ascites protean, are also diagnosed to 25 % of all the patients having chronic liver diseases. Other parameters of ascites, such as ascites pH, carcinoembryonic antigen, lactate dehydrogenase, concentration of lactate and glucoses, lack reliability with respect to distinction between malignant ascites and ascites caused by a non-malignant disease [14]. Prieto M, Gomez-Lechon MJ, Hoyos M, Castell JV, Carrasco D, Berenguer J. Diagnosis of malignant ascites comparison of ascitic fibronectin, cholesterol, serum-ascites albumin difference. Dig Dis Sci 1988; 33 (7): 833-8.

Patients, suffering from accumulation of tissue fluids caused by a neoplastic process are subjected to different treatment compared to when the tissue fluids accumulation is caused by other cases. Correct and timely diagnoses of malignant pleurisy and ascites have significant importance for clinicians. Therefore new methods, enabling accurate diagnoses of malignancy of a tissue fluid are very desirable.

US Patent No. 6,351,667 and US patent application No. 2012/0209085 disclose a method for analyses of liquids, accumulating in the cavities of ascites, pleura and pericardium. But the results of these tests did not achieve considerable improvement in the differential diagnostics of tissue liquids, which accumulation was caused by oncological pathologies [53]. Godie O. Device for detecting pericardial effusion. Patent (No.: US 6,351,667 B1), 2002-02-26; [54]. Degen TW, Thommen DT, Johnson NL. Apparatus and methods for treating intracorporeal fluid accumulation. Patent (No.: US 2012/0209085 A1), 2012-08-16.

Above mentioned patents do not make use of photodynamic diagnostics method.

LT patent No. 5823 and US patent No. 6,860,879 disclose the photodynamic diagnostics method, but in these cases said method is performed in a living person [51]. Bloznelyte-Plesniene L, Mordas A, Liutkeviciu̅te-Navickiene J, Čepulis V, Valuckas KP, Ostapenko V, Rutkovskiene L, Venius J. Intraarterine fotodinamine diagnostika. Patentas (Nr. LT 5823 B), 2012-04-25; [52]. Irion KM, Hahn R. Use of 5-aminolevulinic acid or a derivate thereof for photodynamic diagnosis and/or photodynamic therapy. Patent US 6,860,879 B2, 2005-03-01, where we perform the photodynamic diagnostic tests with ascites, being removed from a person and containing some floating individual cancer cells. Said patents are also related to diagnostics of solid tumors, where our method is intended for the differential diagnostics of tissue fluids caused by oncological and non-oncological pathologies.

European patent application No. 00124107.4 (publ. No. EP 1 203 944 A1) discloses a cancerous cells detection method where a sample of specific naturally existing body fluid is obtained for analysis from a human body after 30-120 min after administering ALA to a patient. Method makes use of rinsing solution and/or body fluids e.g. urine, and/or blood, and/or saliva, and/or vaginal secretion, and/or spermatic fluid, and/or pulmonary output, and/or gastric juice, and/or pancreatic fluid are naturally residing fluids in human body where neither of said body fluids are fluids discharged by a pathological tissue, and where the rinsing solution is not a body fluid but a specially prepared fluid.

There are also many more sources discussing various fluid samples that can be used in various cytological examination procedures for malignancies [61-67].

European patent application No. 05785787.2 (publ. No. EP 1 797 903 A1) discloses use of specific compounds for administration to a human body and later examining samples from a human body in order to verify luminosity of tumor cells. The samples are taken only from tissues and normally existing body fluids.

International patent application No. PCT/JP2011/003509 (publ. No. WO/2011/161933 A1) and European patent application No. 11797822.1 (publ. No. EP 2 584 356 A1) discloses a method for detecting urothelial cancer in a simple manner and with high accuracy, comprising administering 5-aminolevulinic acid (ALA), a derivative thereof, or a salt of the compound or the derivative to a subject, collecting urine from subject, and determining the presence or amount of fluorescence in a cell, contained in the urine collected from subject.

International patent application No. PCT/US2013/044351 (publ. No. WO/2013/184830 A1) discloses methods for detecting brain tumors and assessing the recurrence of such tumors by administering a pharmaceutical composition comprising 5- aminolevulinic acid (5-ALA) and detecting the conversion of 5-ALA to protoporphyrin IX (PPIX) associated with brain-derived microparticles.

There are multiple papers related to application of various photosensitizers for treatment and diagnostics of tumors of different morphological form and various localizations. One of the first experiments related to the research of possibilities of application of photosensitizers for treatment and diagnostics of the malignant tumors were conducted with ascites tumors of test animals, such as Erlich ascites [15]. Musajo L, Visentini P, Bacchinetti F, Razzi MA. Photoinactivation of Ehrlich ascites tumor cells in vitro obtained with skin-photosensitizing furocoumarins. Experientia 1967; 23 (5): 335-6. Another more recent examples on related experiments with animals is paper by Hideki Sakurai et al., titled "Basic study on photodynamic therapy in liver cell carcinoma with reference to experimental liver tumor in rabbits and distribution of photosensitive PH-1126" [55]. The paper discloses photodynamic diagnosis combined with photosensitive substance PH-1126 for detecting liver tumor cells in blood. And paper by Flvia Rodrigues de Oliveira Silva et al., titled Enhancement of blood porphyrin emission intensity with aminolevulinic acid administration: A new concept for photodynamic diagnosis of early prostate cancer" discloses oral administration of ALA in animals with prostate tumor to increase the sensitivity of cancer diagnoses by protoporphyrin IX blood auto fluorescence [56]. This method is directed to analyzing blood for cancer cells with absorbed δ-aminolevulinic acid (ALA). Nevertheless neither these papers nor any other accessible scientific paper discloses identification of tissue fluid malignancy level or differential diagnostics to determine whether an accumulation of tissue liquids is caused by an oncological disease or a non-malignant pathology [16]. Sibani SA, McCarron PA, Woolfson AD, Donnelly RF. Photosensitiser delivery for photodynamic therapy. Part 2: systemic carrier platforms. Expert Opin Drug Deliv 2008; 5 (11): 1241-54. [17]. El-Far M, Elshal M, Bondock S, Refaat M. Preclinical biochemical studies using a novel 5-aminolevulinic acid ester derivative with superior properties for photodynamic therapy of tumors. Int J Med Medic Sci 2009; 1 (7): 278-87. [18]. Smolka J, Mateasik A, Cunderlikova B, Sanislo L, Mlkvy P. In vivo fluorescence diagnostics and photodynamic therapy of gastrointestinal superficial polyps with aminolevulinic acid. A clinical and spectroscopic study. Neoplasma 2006; 53 (5): 418-23. [19]. Borisova E, Vladimirov B, Avramov L. 5-ALA/PpIX fluorescence detection of esophageal and stomach neoplasia - effects of autofluorescence background from normal and inflammatory areas. Proc SPIE 2008; 7027: 70271A-1-9. [20]. Stummer W, Stocker S, Wagner S, Stepp H, Fritsch C, Goetz C, et al. Intraoperative detection of malignant gliomas by 5-aminolevulinic acid-induced porphyrin fluorescence. Neurosurgery 1998; 42 (3): 518-526. [21]. Rydell R, Eker C, Andersson-Engels S, Krogdahl A, Wahlberg P, Svanberg K. Fluorescence investigations to classify malignant laryngeal lesions in vivo. Head Neck 2008; 30: 419-26. [22]. Chatterton K, Ray E, O'Brien TS. Fluorescence diagnosis of bladder cancer. Br J Nurs 2006; 15: 595-7. [23]. Pham W. Detection and treatment of diseases using light. IFMBE Proc 2010; 27: 19-22. [24]. Scott MA, Hopper C, Sahota A, Springett R, McIlroy BW, Bown SG, et al. Fluorescence photodiagnostics and photobleaching studies of cancer lesions using ratio imaging and spectroscopic techniques. Lasers Med Sci 2000; 15: 63-72. [25]. Andersson-Engels S, Klinteberg C, Svanberg K, Svanberg S. In vivo fluorescence imaging for tissue diagnostics. Phys Med Biol 1997; 42: 815-24. [26]. Wang I, Clemente LP, Pratas RMG, Cardoso E, Clemente MP, Montan S, et al. Fluorescence diagnostics and kinetic studies in the head and neck region utilizing low-dose δ-aminolevulinic acid sensitization. Cancer Lett 1999; 135: 11-9. [27]. Ackroyd R, Kelty C, Brown N, Reed M. The history of photodetection and photodynamic therapy. Photochem Photobiol 2001; 74 (5): 656-69. [28]. Burger M, Zaak D, Stief CG, Filbeck T, Wieland WF, Roessler W, et al. Photodynamic diagnostics and noninvasive bladder cancer: is it cost-effective in long-term application? A Germany-based cost analysis. Eur Urol 2007; 52: 142-7. [29]. Hillemanns P, Reiff J, Stepp H, Soergel P. Lymph node metastasis detection of ovarian cancer by porphyrin fluorescence photodetection: case report. Lasers Med Sci 2007; 22: 131-5. [30]. Bloznelyte-Plesniene L, Rutkovskiene L. Photodynamic therapy of malignant and benign tumours in Lithuania. Acta medica Lituanica 2007; 14 (3): 193-200. [31]. Liutkeviciute-Navickiene J, Mordas A, Rutkovskiene L, Bloznelyte-Plesniene L. Skin and mucosal fluorescence diagnosis with different light sources. Eur J Dermatol 2009; 19 (2): 135-40. [32]. Rigual NR, Thankappan K, Cooper M, Sullivan MA, Dougherty T, Popat SR, et al. Photodynamic therapy for head and neck dysplasia and cancer. Arch Otolaryngol Head Neck Surg 2009; 135 (8): 784-8. [33]. Schweitzer VG, Somers ML. Photofrin-mediated photodynamic therapy for treatment of early stage (Tis-T2N0N0)SqCCa of oral cavity and oropharynx. Lasers Surg Med 2010; 42: 1-8. [34]. Canavesi C, Fournier F, Cassarly WJ, Foster TH, Rolland JP. Illumination devices for photodynamic therapy of the oral cavity. Biomed Opt Express 2010; 1 (5): 1480-90. [35]. Karakullukcu B, Oudenaarde K, Copper MP, Klop WMC, Veen R, Wildeman M, et al. Photodynamic therapy of early stage oral cavity and oropharynx neoplasms: an outcome analysis of 170 patients. Eur Arch Otorhinolaryngol 2011; 268: 281-8. [36]. Meissner O, Ostermeier M, Hoheisel M, inventors. Combined OCT catheter device and method for combined optical coherence tomography (OCT) diagnosis and photodynamic therapy (PDT). United States patent US 20090240154. 2009 Sep 24. [37]. Zenk W, Dietel W, Schleier P, Gunzel S. [Visualizing carcinomas of the mouth cavity by stimulating synthesis of fluorescent protoporphyrin IX]. Mund Kiefer Gesichtschir 1999; 3 (4): 205-9. [38]. Lane PM, Gilhuly T, Whitehead P, Zeng H, et al. Simple device for the direct visualization of oral-cavity tissue fluorescence. J Biomed Opt 2006; 11: 024006 1-7. [39]. Toursounidis T, Upile Ch, Betz S, Shah P, et al. Fluorescence spectroscopy in the detection of oral dysplasia. Head Neck Oncol 2009; 1 (Suppl 1): P3. [40]. Heintzelman DL, Utzinger U, Fuchs H, et al. Optimal excitation wavelengths for in vivo detection of oral neoplasia using fluorescence spectroscopy. Photochem Photobiol 2000; 72 (1): 103-13. [41]. Smith PW. Fluorescence emission-based detection and diagnosis of malignancy. J Cellul Biochem Suppl 2002; 39: 54-9. [42]. Pathak N, Davis L, Hsiang YN, et al. Detection of squamous neoplasia by fluorescence imaging comparing porfimer sodium fluorescence to tissue autofluorescence in the hamster cheek-pouch model. Am J Surg 1995; 170: 423-6. [43]. Westra WH, Sidransky D. Fluorescence visualization in oral neoplasia: shedding light on an old problem. Clin Cancer Res 2006; 12 (22): 6594-7. [44]. Friesen SA, Hjortland GO, Madsen SJ, Hirschberg H, Engebraten O, Nesland JM, Peng Q. 5-aminolevulinic acid-based photodynamic detection and therapy of brain tumors. Int J Oncol 2002; 21: 577-82. [45]. Tope WD, Ross EV, Kollias N, Martin A, et al. Protoporphyrin IX fluorescence induced in basal cell carcinoma by oral delta-aminolevulinic acid. Photochem Photobiol 1998; 67: 249-55. [46]. Johansson J, Berg R, Svanberg K, Svanberg S. Laser-induced fluorescence studies of normal and malignant tumour tissue of rat following intravenous injection of delta-amino levulinic acid. Lasers Surg Med 1997; 20: 272-9. [47]. Rick K, Sroka R, Stepp H, Kriegmair M, et al. Pharmacokinetics of 5-aminolevulinic acid-induced protoporphyrin IX in skin and blood. J Photochem Photobiol B 1997; 40: 313-9. [48]. Leunig A, Rick K, Stepp H, Gutmann R, et al. Fluorscence imaging and spectroscopy of 5-aminolevulinic acid induced protoporphyrin IX for the detection of neoplastic lesions in the oral cavity. Am J Surg 1996; 172: 674-7. [49]. Campbell DL, Gudgin-Dickson EF, Forkert PG, et al. Detection of early stages of carcinogenesis in adenomas of murine lung by 5-aminolevulinic acid-induced protoporphyrin IX fluorescence. Photochem Photobiol 1996; 64: 676-82. [50]. Gronlund-Pakkanen S, Makinen K, Talja M, Kuusisto A, Alhava E. The importance of fluorescence distribution and kinetics of ALA-induced PpIX in the bladder in photodynamic therapy. J Photochem Photobiol B 1997; 438: 269-73. Malini Olivo et al: "A new frontier in hypericin-mediated diagnosis of cancer with current optical technologies", Annals of biomedical engineering, Kluwer academic publishers-plenum publishers, NE, vol. 40, no.2, 29 November 2011, pages 460-473 [57]. Pytel Akos et al.: "New aspect of photodynamic diagnosis of bladder tumors: Fluorescence cytology", Urology, Belle Mead, NJ, US, vol. 59, no. 2, 1 February 2002, pages 216-219 [58]. Stephan Tauber et al: "Fluorescence cytology of the urinary bladder", Urology, vol. 61, no. 5, 1 May 2003, pages 1067-1071 [59]. Stephan Tauber et al: "Integral spectrophotometric analysis of 5-aminolaevulinic acid-induced fluorescence cytology of the urinary bladder", BJU international, vol. 97, no. 5, 1 May 2006, pages 992-996 [60].

### Brief description of the invention

The invention is related to a fairly simple method of a non-invasive diagnostics of tissue liquids caused by an oncological pathology, in particular to the photodynamic diagnostics of the malignant tissue liquids.

This method comprises a systematic injection of a photosensitizer (derivative of hematoporphyrin or other photosensitizer). A liquid is punctured after a time interval which is required for the photosensitizer to accumulate selectively in the tumor tissue. The tissue liquid which was obtained during a puncture is illuminated with a non-intensive 400-405 nm violet light. If an accumulation of tissue liquid was caused by an oncological pathology, then the liquid will contain floating individual tumor molecules. Even a small amount of the malignant tissue cells in a tissue liquid will produce a vivid raspberry-color glow of said fluid. Tissue fluids of another origin do not have this king of glow when illuminated with the light having 400-405 nm wavelengths. According to our data this test has sensitivity of 96 % and specificity of 100 %. The test is sensitive even when tumor cells are not found during a cytological analysis. One drop of tissue liquid is sufficient for the test to be performed. The invention is based on a fairly selective accumulation of photosensitizers in all the malignant tissues and all cells of the malignant tumors [23, 30, 31, 41, 44, 46]. Individual tumor cells, floating in an accumulation of tissue fluid caused by the malignant tumors, before detachment from tumor hotbeds also have certain amount of photosensitizers. Thus when a patient diagnosed with an oncological disease which causes an accumulation of tissue liquids is systematically administered with a photosensitizer into vein or artery or orally (other ways of systematic administration are also possible), after some time depending on the composition of the photosensitizer, method administration and dose said photosensitizer will fairly selectively accumulate in tumor cells. The photosensitizer will also selectively accumulate in tumor cells, which pass to a tissue liquid. When tumor cells having sufficient amount of photosensitizer are illuminated the characteristic glow can be observed using proper excitation light to illuminate the photosensitized tumors during diagnostic. In the case of porphyritic photosensitizers the light of 400-405 nm wavelengths is used. It is also possible to use a wider 380 - 410 nm range of the activating light. When choosing a range of wavelengths of a light it is important to take into account properties of the used photosensitizer. The malignant tumor tissues having accumulated the porphyritic photosensitizers emit an intensive glow when illuminated by 400-405 nm light, where said glow is visible for a naked eye, has the characteristic raspberry-color and can be photographed. Using a spectrometer it is possible to evaluate intensity of the glow and to perform its spectroscopic analyses. The malignant tumor cells which are present in a tissue liquid emit an intensive glow when illuminated with 400-405 nm light, where the glow is visible for a naked eye and has the characteristic raspberry-color. After sufficient time has passed after the systemic administration of a sensitizer, in particular after 24 hours after an intravenous administration of a derivative of hematoporphyrin, in particular the photofrin (2 mg/kg), photohem (2.5 mg/kg), photosan (2 mg/kg), after puncture of tissue liquids (of pleurisy, ascites, and others) of the malignant tumors and subsequent storage in a clear container, such as a syringe, test-tube or a bag for collection of tissue liquid, and illumination with 400-405 nm light, the raspberry-color glow will be observed. If the cause of accumulation of tissue liquid will be a non-malignant pathology, the liquid will be tumor-cells-free, as well as it will not produce said glow even if it will contain admixtures of blood (haemorrhagic ascites or pleurisy). The test requires only one drop of a tissue liquid.

### Brief description of drawings

- Fig. 1: The pleural liquid in a collection bag. Pleural liquid caused by the squamous cell cancer.
- Fig. 2: A light emitting diode.
- Fig. 3: A pathological pleural liquid in a collection bag, illuminated with a violet light. Pleural liquid was caused by a squamous cell lung cancer. The characteristic raspberry-color glow is present.
- Fig. 4: An ascites in a liquid collection bag. Ascites was caused by an outspread of the colon cancer.
- Fig. 5: An ascites illuminated by a violet light in a collection bag. Ascites was caused by an outspread of the colon cancer. The characteristic raspberry-color glow is present.
- Fig. 6: A punctate of an ascites (middle test-tube is illuminated with a violet light in a 2 ml test-tube). Ascites is caused by the colon adenocarcinoma. Lateral test-tubes contain tissue liquids caused by a non-malignant pathology. The glow is not observed.

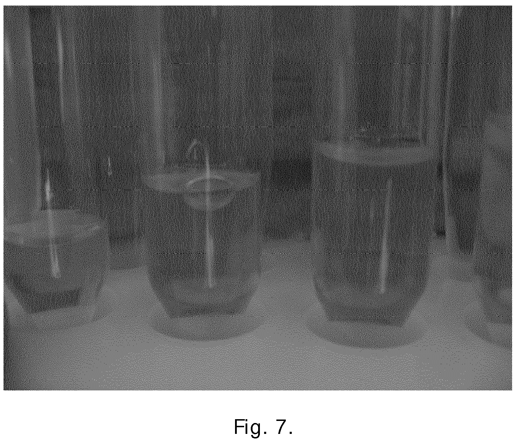

- Fig. 7: Tissue liquids caused by a non-malignant pathology, illuminated by a violet light. The glow is not observed.

### Detailed description of the invention

### Material and methodology

Our photodynamic diagnostics method can make use of various photosensitizers. We have used the following ones:
1. Photofrin® 75, Axcan Pharma SAS, France. This is a derivative of hematoporphirin. 75 mg of darkish red-brown powder of Photofrin is distributed in a sterile glass bottles.
2. Photogemum, 0,2 g (OOO Photogem, Moscow, Russia), analogue,

Fluorescent excitation is achieved with 405±5 nm wavelength light. The fluorescence was monitored with a naked eye and registered by an optical spectrometer QE65000 (Ocean Optics).

For patients suffering from accumulation of tissue liquids, e.g. ascites or liquid in pleural cavity, a frequent puncturing is performed to diagnose a disease, which has caused accumulation of tissue liquid in pleural or peritoneal cavity, also for treatment purpose to ease condition of a patient by extracting a pathological substance from said cavity. When performing puncturing of a tissue liquid a body cavity wall is pierced with a special needle or a trocar.

Sometimes drainage of tissue liquids is performed for this type of patients.

### Typical aspiration puncturing of pleural cavity

Indications: determining composition of liquid (exudate or transudate) in a pleural cavity; it's cytological, biochemical, bacteriological and other tests are performed for purposes of differential diagnostics.

Execution methodology. Puncturing is performed in a gap between the VI - VII ribs, in a projection of the frontal or back line of underarm, along the upper edge of the lower rib. During the procedure skin is cleaned with antiseptics, the soft tissues of the chest wall, if necessary for drainage of pleural cavity, are sufficiently infiltrated with local anesthetic solution. A disposable system is used, which is connected with a puncturing needle and a syringe by a trident. The near wall pleura is punctured near the edge of the upper rib. Liquid or air is extracted in slow pace in order for the internal organs to have sufficient time to adapt to decreasing pressure. It is not advisable to perform a complete extraction of said liquid from a pleura cavity during one procedure to avoid increasing risk of complications. It is recommended to extract no more than 1 l of the liquid per one procedure.

If indications are present, drainage of pleural cavity is performed.

Method of drainage:
1. Using a trocar: skin is cleaned with an antiseptic; the selected zone is injected with an anesthetic; a U form stich is made through skin/hypoderm and a 1 - 1.5 cm cut is made in the middle of the U form stitch; the chest wall is pierced by a trocar and stiletto is removed. Drain is inserted into pleural cavity, trocar tube is removed and drain is fixed to the skin/hypoderm by ligatures. Other end of the drain is connected to the reservoir for discharged fluids.
2. Without trocar: skin is cleaned with an antiseptic; the selected zone is injected with an anesthetic; a 1 - 1.5 cm cut is made in the selected zone, a blunt instrument is used to part the muscles; the near-wall pleura is carefully pierced; drain is inserted; fixed to the skin. Other end of the drain is connected to the reservoir for discharged fluids.

Typical puncture of ascites of peritoneum cavity (paracentesis). Indications:
1. In order to determine diagnoses in case of ascites of an unknown origin.
2. For assessment of possible peritonitis.
3. In order to alleviate abdomen pain in case of intense ascites.
4. In order to alleviate breathing when diaphragm is lifted because of ascites.
5. For assessment of an abdomen bleeding after a blunt trauma in the abdomen.
6. Peritoneal dialyses.

### Preparation of a patient for the procedure:

Patient is required to urinate. Although sometimes is necessary, catheterization with a folic catheter is not recommended. Location of paracentesis is determined: the straight abdominal muscle is located. Best point for puncture is to the side by 2 - 3 cm from the edge of *m. rectus abdominis*, in the lower quadrants of an abdomen. Alternative location for puncture is Linea alba, located 3 - 4 cm lower of a navel.

When choosing location for puncture, following has to be avoided:
1. The straight abdominal muscles (increased risk of bleeding from epigastric blood vessels).
2. Surgical scars (increased risk of intestine perforation due to accretion of intestines to the walls of abdomen.
3. Inflammatory zones of a skin (increased risk of infection).

Execution methodology: the puncture area is cleaned with an antiseptic, a blanket is used for covering. The puncture zone is anesthetized with a solution of 1 % or 2 % lidocain. A puncture needle is introduced vertically with respect to the wall of an abdomen. Small cracking sensation is felt when fascia of muscles is punctured, and a sudden pull is felt after the needle makes its way into the abdomen (it is important to avoid sudden onward moves). Z puncture technique can be used for prophylaxis of percolation of the liquid - after the needle pierces the skin it is moved away to avoid direct piercing of the abdomen cavity. Necessary volume of the liquid is removed (usually not more than 1 liter). If puncturing is intended for purpose of diagnostics, angiocatheter, which covers the needle during the procedure, can be used. Such catheter can be somewhat moved when flow of the liquid stops. Transfusion with albumins is necessary after more than 4 - 5 l of said liquid is removed to avoid losing proteins. If paracenthesis is performed for purpose of diagnostics, material for the test is taken from the punctured ascites: 1) to determine quantity of LDH, glucoses, and albumin; 2) to determine density of proteins and specific ascites. For the cytological, bacteriological, diagnostics of carcinoembryonic antibody and other tests. It is convenient to perform both the tissue liquid puncture and drainage under control of an ultrasound. By applying photodynamic diagnostics for a patient with tissue liquids caused by an oncological pathology photosensitizer are introduced before performing the puncture of tissue liquid. Time interval from the moment of introduction of sensitizer until performing puncture or drainage depends on characteristics of a sensitizer, its introduction method, e.g. intravenous, oral or other, also doze of a sensitizer.

Using derivatives of hematoporphyrin for photodynamic diagnostics and administering them intravenously optimal doze of pharmaceutical is: 2 mg of Photofrin per kg of weight, 2 mg of photosan per kg of weight, 2.5 mg of photohem per kg of weight. Test results of a tissue liquid punctate are conclusive after 24 h after intravenous injection of the preparation and remains such for 144 h. Later on, depending on individual traits of a patient, conclusiveness of a photodynamic diagnostics decreases.

Punctate sample of the tissue liquid is placed in a test-tube or other receptacle with clear walls (e.g. the syringe, which is used for puncturing) and subsequently subjected to the 405 nm violet light (possible range of wavelengths of activating light is 380 - 410 nm). It is important to take into account the chosen sensitizer when selecting the range of wavelengths of light. The test requires only one drop of a tissue liquid for illumination with violet light.

We use violet light emitting LEDs as the light source. If a malignant tumor cells are present in a tissue liquid it will glow, where said glow will be intensive, visible with naked eye, and will have the characteristic raspberry-color when illuminated with light, having 400 - 405 nm wavelength, wherein intensity of the glow correlates with HpD dose, histological structure of the tissue, and structure of the cells.

From year 1989 we have treated more than 1500 oncological patients with the sensitized tumor therapy by intravenous injection of derivatives of hematoporphyrin or by administering a solution of ALA derivatives by drinking (6 patients). Amongst the patients 32 subjects received puncturing of tissue liquids from various cavities, whereby extracted puncture liquid was subjected to the photodynamic diagnostics, where: 12 patients received pleural puncturing and drainage, 12 patients received puncturing of ascites and drainage, 1 patient received puncturing of pericardia, 2 patients received puncturing of the non-malignant neck cysts, 5 patients received puncturing of tissue liquids of metastases of the collapsing tumors in a neck zone. Data on said 32 subjects is given in a table below:

| **Patient** | | **Histology of tumour** | **Puncture** | | **FD diagnostics , glow: yes/no** |
|---|---|---|---|---|---|
| **No** | **Diagnoses** | | **Location** | **Origin (oncopathology ,yes, no)** | |
| 1. | Lung cancer | Squamous ca | pleura | **oncopathology** | Yes |
| 2. | Lung cancer | Squamous ca | pleura | **oncopathology** | Yes |
| 3. | Lung cancer | Squamous ca | pleura | **oncopathology** | Yes |
| 3 | Lung cancer | Squamous ca | Neck cysta | **no** | No |
| 4. | Lung cancer | Adenocarcinoma | pleura | **oncopathology** | Yes |
| 5. | Lung cancer | Adenocarcinoma | pleura | **oncopathology** | Yes |
| 6. | Lung cancer | Small cell ca | pleura | **oncopathology** | Yes |
| 7. | Lung cancer | Small cell ca | Pleura | **oncopathology** | Yes |
| 7 | Lung cancer | Small cell ca | Pericardium | **oncopathology** | Yes |
| 8. | Breast cancer | Adenocarcinoma | Pleura | **oncopathology** | Yes |
| 8 | Breast cancer | Adenocarcinoma | Seroma in armpit | **no** | No |
| 9. | Breast cancer | Adenocarcinoma | Pleura | **oncopathology** | Yes |
| 10. | Liver Cholangiocarcinoma | Cholangiocarcinoma | Pleura | **oncopathology** | Yes |
| 10 | Liver Cholangiocarcinoma | Cholangiocarcinoma | Peritoneal cavity | **oncopathology** | Yes |
| 11. | Stomach cancer | Adenocarcinoma | Pleura | **oncopathology** | Yes |
| 12. | Large intestine cancer | Adenocarcinoma | Pleura | **oncopathology** | Yes |
| 12 | Large intestine cancer | Adenocarcinoma | Peritoneal cavity | **oncopathology** | Yes |
| 13. | Colorectal cancer | Adenocarcinoma | Peritoneal cavity | **oncopathology** | Yes |
| 14. | Thyroid cancer | Adenocarcinoma | Thyroid cyst | **no** | No |
| 15. | Stomach cancer | Adenocarcinoma | Peritoneal cavity | **oncopathology** | Yes |
| 16. | Stomach cancer | Adenocarcinoma | Peritoneal cavity | **oncopathology** | Yes |
| 17. | Colorectal cancer | Adenocarcinoma | Peritoneal cavity | **oncopathology** | Yes |
| 18. | Colorectal cancer | Adenocarcinoma | Peritoneal cavity | **oncopathology** | Yes |
| 19. | Colorectal cancer | Adenocarcinoma | Peritoneal cavity | **oncopathology** | Yes |
| 20. | Prostate cancer | Prostate carcinoma | Peritoneal cavity | **oncopathology** | Yes |
| 21. | Skin Melanoma, mts in liver | Melanoma | Peritoneal cavity | **oncopathology** | No |
| 22. | Kidney Cancer | Kidney light-cell carcinoma | Peritoneal cavity | **oncopathology** | Yes |
| 23. | Osteogenic sarcoma | sarcoma | Elbow joint | **oncopathology** | Yes |
| 24. | Leomiosarcoma | sarcoma | Peritoneal cavity | **oncopathology** | Yes |
| 25. | Laryngeal cancer | Squamous ca | Collapsing giant Mts in a neck | **oncopathology** | Yes |
| 26. | Oropharyngeal cancer | Squamous ca | Collapsing giant Mts in a neck | **oncopathology** | Yes |
| 27. | Oropharyngeal cancer | Squamous ca | Neck cysta | **no** | No |
| 28. | Jujing sarcoma | Sarcoma | Knee joint | **no** | No |
| 29. | Oropharyngeal cancer | Squamous ca | Post-operation Seroma | **no** | No |
| 30. | Cervical Cancer | Squamous ca | Groin postoperative seroma | **no** | No |
| 31. | Breast cancer | Adenocarcinoma | Seroma in an armpit | **no** | No |
| 32. | Kidney cancer | Light-cell carcinoma | Cyst in soft tissue | **no** | No |

1 patient was punctured in the knee joint for liquids, which had accumulated because of a sarcoma, 7 patients were punctured in the stasis tissue liquid (for 4 in armpit), caused by postoperative stasis phenomenon. From the 32 patients, that received puncture of tissue liquids and subsequent analysis of said tissue liquids, in 7 subjects puncture was performed for a non-malignant pathology caused tissue liquids, in 23 subjects puncture was performed for oncological pathology caused tissue liquids, and for 2 subjects puncture was performed for both a non-malignant and oncopathology caused tissue liquids.

In 24 patients the tissue liquid caused by an oncopathology had the characteristic glow when subjected to the photodynamic diagnostics. In one patient the ascites caused by a melanoma did not have the characteristic glow when subjected to the photodynamic diagnostics. (We have established that photodynamic diagnostics is not effective for melanoma, after photodynamic diagnostics was applied for malignant tumors of different morphological forms [30, 31]). Also no glow was observed in photodynamic diagnostics which was applied for tissue liquids caused by a non-malignant pathology. A non-malignant pathology caused tissue liquids, from 2 patients, under FDD did not have the characteristic glow, but oncopathology caused tissue liquids, from same patients, had the glow. For the glow to be observable, only 1 drop of tissue liquid on a test glass was sufficient.

In general, it is evident, that the photodynamic diagnostics has sensitivity of 96 % and specificity of 100 %, when FDD is applied for tissue liquids caused by an oncological pathology. Because no glow was observed only in the case of ascites caused by a melanoma, by applying FDD to tissue liquids caused by the non-melanomic oncopathology its sensitivity and specificity is considered to be 100 %. Data is subjected for variation when number of test subjects increases.

Further on for illustrative purposes we will provide examples, which should not be considered as constraining scope of the invention.

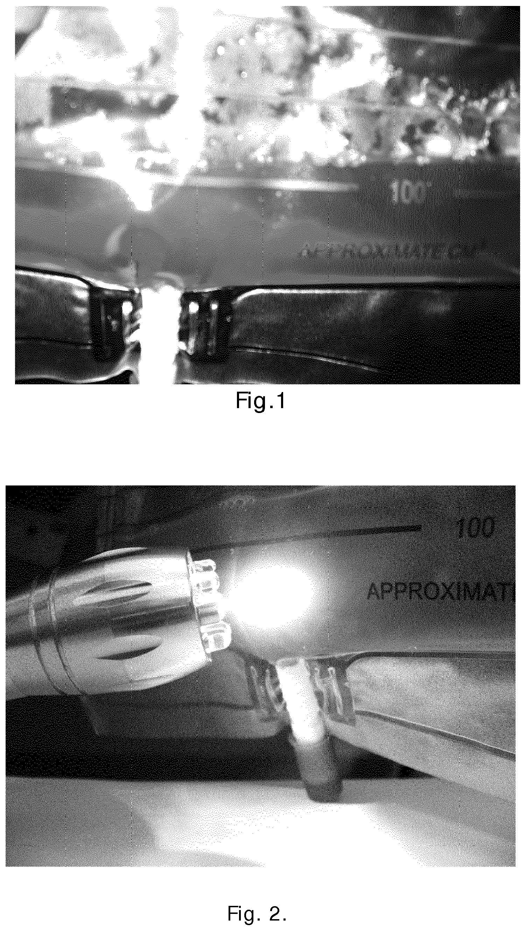

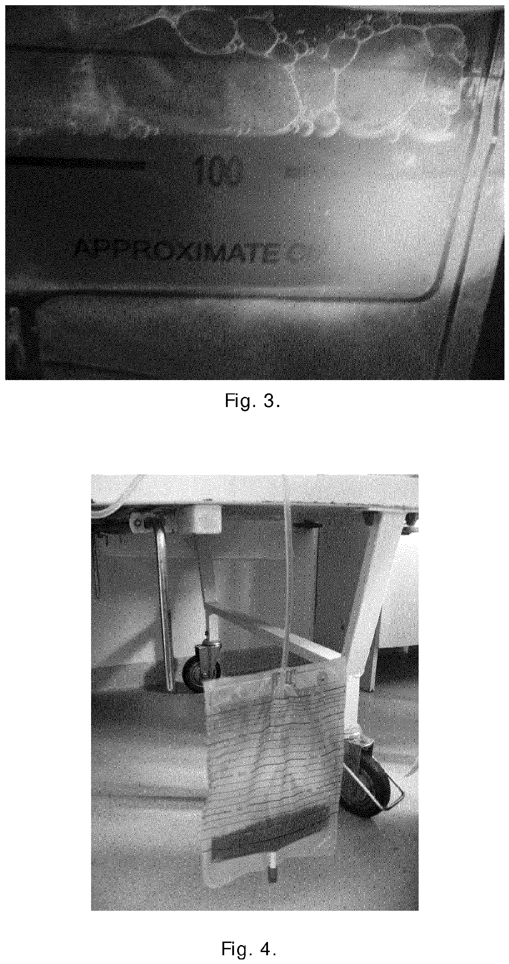

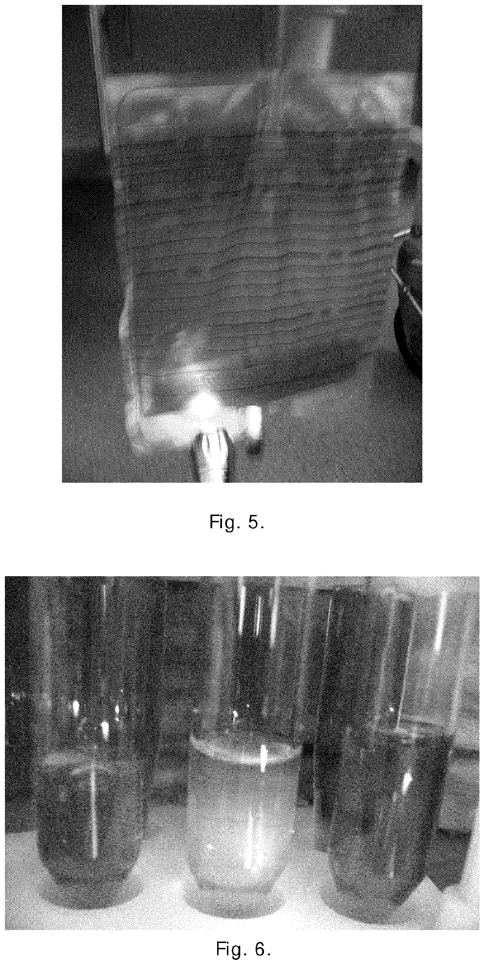

Example No. 1: An outspreaded squamous cell carcinoma of a patient was treated with an intravenous photodynamic therapy by using photohem, a derivative of hematoporphyrin. 2.5 mg of photohem per kg of weight, diluted with 100 ml of physiological solution of NaCl, was injected. After 24 h tumor in the left bronchus was illuminated with a red laser light through an endoscope. Wavelength of the light was 630 nm, power - 100 mW. 72 hours after the injection of photohem, accumulation of a liquid in a cavity of pleura was punctured and 1 l of said liquid was drawn out (Fig. 1 is liquid of pleura in liquid accumulation bag). Cancer cells were detected by a cytological test. The raspberry-color glow (Fig. 3) was observed after liquid accumulation bag filled with pleural liquid was illuminated with a violet light of a light emitting diode (Fig. 2). The intensive raspberry-color glow (Fig. 6, middle test-tube) was observed when 10 ml of said liquid in the test tube was subjected to the violet light of 405 nm and 2 mW power LED.

Same patient was also diagnosed a neck cyst. 72 hours after the injection of photohem, 10 ml of liquid was drained into a test tube by puncture and illuminated with the violet light of 405 nm and 2 mW power LED - no glowing was observed (Fig. 6, lateral test-tubes).

Example No. 2: Large intestine glandular outspreaded cancer of a patient was treated with an intravenous therapy of sensitized tumors by using photohem, a derivative of hematoporfirin. 2.5 mg of photohem per kg of weight, diluted with 100 ml of a physiological solution of NaCl, was injected. A drain was inserted into the patient because of accumulation of ascites in peritoneum. A special bag for accumulation of liquid was attached to the drain (Fig. 4). After 24 h after the injection of photohem, the bag was illuminated with the 405 nm violet light from diodes, after which the intensive raspberry-color glow was observed (Fig. 5). In a period of 144 hours ascites in the patient was constantly drained because fresh ascites was replenishing itself. Said drained ascites in the period of 144 h had the intensive raspberry-color glow at any time during said period, when illuminated with 2mW power LEDs, emitting the 405 nm wavelength violet light. After the 144 h have passed intensity of the raspberry-color glow started to diminish rapidly. In order to observe the glow during the 144 h period, 1 drop of tissue liquid was sufficient for the test on a test glass. Approach of contemporary diagnostics methods for diagnoses of an accumulation of tissue liquids caused by an oncological pathology is rather limited. Although specificity of morphological tests for tissue liquids caused by an oncological diseases is close to 100 %, but the sensitivity is poor. Sensitivity of a cytological test for diagnoses of ascites caused by an oncological pathology, according to data of different authors is only 40 - 60 %. Sensitivity of a cytological test for diagnoses of an accumulation of tissue liquids in pleura, caused by oncological pathology, is only 60 %. Biopsy of pleura increases sensitivity of morphological tests. But even if it is performed during a computer tomography, only 87 % of all patients are diagnosed precisely, which means that sensitivity is increased to 87 %. Other test indicators like high level of proteins of ascites, characteristic to the malignant ascites, are diagnosed to 25 % of all patients, having chronic liver diseases. Other parameters, like ascites pH, carcinoembrionic antigen, lactate dehydrogenase, concentration of lactate and glucoses are insufficiently reliable for distinguishing ascites caused by the malignant diseases and the non-malignant diseases. Specificity of theses test does not exceed 75 %. Similarly, specificity of other tests for pleural liquids also does not exceed 75 %. Sensitivity and specificity of our photodynamic diagnostics method for tissue liquids caused by oncological pathologies is 96 % and 100 %, respectively. According to our data this method is sensitive and specific when a malignant tissue liquid is caused by malignant tumors of different morphological types, except for melanoma. Test requires only one drop of tissue liquid.

### References

1. Lombardi G, Zustovich F, Nicoletto MO, Donach M, Artioli G, Pastorelli D. Diagnosis and treatment of malignant pleural effusion: a systematic literature review and new approaches. Am J Clin Oncol 2010; 33 (4): 420-3.
2. Zablockis R, Nargėla R. Pleuros skysčio citologinio tyrimo diagnostinė reikšmė. Medicina 2002; 38 (12): 1171-8.
3. Marel M, Zrustova M, Stasny B, Light RW. The incidence of pleural effusion in a well-defined region: epidemiologic study in central Bohemia. Chest 1993; 104 (5): 1486-9.
4. Valdes L, Alvarez D, Valle JM, Pose A, San Jose E. The etiology of pleural effusions in an area with high incidence of tuberculosis. Chest 1996; 109 (1): 158-62.
5. Inan I, De Sousa S, Myers PO, Bouclier B, Dietrich PY, Hagen ME, Morel P. Management of malignant pleural effusion and ascites by a triple access multi perforated large diameter catheter port system. World J Surg Oncol 2008; 6: 85. doi: 10.1186/1477-7819-6-85
6. Johnston WW. The malignant pleural effusion. A review of cytopathological diagnoses of 584 specimens from 472 consecutive patients. Cancer 1985; 56 (4): 905-9.
7. Rodriguez-Panadero F, Romero-Romero B. Management of malignant pleural effusions. Curr Opin Pulm Med 2011; 17: 269-73.
8. Rodriguez-Panadero F, Borderas Naranjo F, Lopez-Mejias J. Pleural metastatic tumours and effusions. Frequency and pathogenic mechanisms in a post-mortem series. Eur Respir J 1989; 2 (4): 366-9.
9. Chernow B, Sahn SA. Carcinomatous involvement of the pleura: an analysis of 96 patients. Am J Med 1977; 63: 695-702.
10. Bille A, Borasio P, Gisabella M, Errico L, Gatherer R, Ardissone F. Suitable device for thoracoscopic talc poudrage in malignant pleural effusion. Gen Thorac Cardiovasc Surg 2011; 59 (7): 522-4.
11. Cheong JC, Choi WH, Kim DJ, Park JH, Cho SJ, Choi CS, Kim JS. Prognostic significance of computed tomography defined ascites in advanced gastric cancer. J Korean Surg Soc 2012; 82: 219-26.
12. Eskander RN, Tewari KS. Emerging treatment options for management of malignant ascites in patients with ovarian cancer. Int J Womens Health 2012; 4: 395-404.
13. Grannis Jr. FW, Lai L, Kim JY. Fluid complications. Cancer Management: 14th edition 2011.
14. Prieto M, Gomez-Lechon MJ, Hoyos M, Castell JV, Carrasco D, Berenguer J. Diagnosis of malignant ascites comparison of ascitic fibronectin, cholesterol, serum-ascites albumin difference. Dig Dis Sci 1988; 33 (7): 833-8.
15. Musajo L, Visentini P, Bacchinetti F, Razzi MA. Photoinactivation of Ehrlich ascites tumor cells in vitro obtained with skin-photosensitizing furocoumarins. Experientia 1967; 23 (5): 335-6.
16. Sibani SA, McCarron PA, Woolfson AD, Donnelly RF. Photosensitiser delivery for photodynamic therapy. Part 2: systemic carrier platforms. Expert Opin Drug Deliv 2008; 5 (11): 1241-54.
17. El-Far M, Elshal M, Bondock S, Refaat M. Preclinical biochemical studies using a novel 5-aminolevulinic acid ester derivative with superior properties for photodynamic therapy of tumors. Int J Med Medic Sci 2009; 1 (7): 278-87.
18. Smolka J, Mateasik A, Cunderlikova B, Sanislo L, Mlkvy P. In vivo fluorescence diagnostics and photodynamic therapy of gastrointestinal superficial polyps with aminolevulinic acid. A clinical and spectroscopic study. Neoplasma 2006; 53 (5): 418-23.
19. Borisova E, Vladimirov B, Avramov L. 5-ALA/PpIX fluorescence detection of esophageal and stomach neoplasia - effects of autofluorescence background from normal and inflammatory areas. Proc SPIE 2008; 7027: 70271A-1-9.
20. Stummer W, Stocker S, Wagner S, Stepp H, Fritsch C, Goetz C, et al. Intraoperative detection of malignant gliomas by 5-aminolevulinic acid-induced porphyrin fluorescence. Neurosurgery 1998; 42 (3): 518-526.
21. Rydell R, Eker C, Andersson-Engels S, Krogdahl A, Wahlberg P, Svanberg K. Fluorescence investigations to classify malignant laryngeal lesions in vivo. Head Neck 2008; 30: 419-26.
22. Chatterton K, Ray E, O'Brien TS. Fluorescence diagnosis of bladder cancer. Br J Nurs 2006; 15: 595-7.
23. Pham W. Detection and treatment of diseases using light. IFMBE Proc 2010; 27: 19-22.
24. Scott MA, Hopper C, Sahota A, Springett R, McIlroy BW, Bown SG, et al. Fluorescence photodiagnostics and photobleaching studies of cancer lesions using ratio imaging and spectroscopic techniques. Lasers Med Sci 2000; 15: 63-72.
25. Andersson-Engels S, Klinteberg C, Svanberg K, Svanberg S. In vivo fluorescence imaging for tissue diagnostics. Phys Med Biol 1997; 42: 815-24.
26. Wang I, Clemente LP, Pratas RMG, Cardoso E, Clemente MP, Montan S, et al. Fluorescence diagnostics and kinetic studies in the head and neck region utilizing low-dose δ-aminolevulinic acid sensitization. Cancer Lett 1999; 135: 11-9.
27. Ackroyd R, Kelty C, Brown N, Reed M. The history of photodetection and photodynamic therapy. Photochem Photobiol 2001; 74 (5): 656-69.
28. Burger M, Zaak D, Stief CG, Filbeck T, Wieland WF, Roessler W, et al. Photodynamic diagnostics and noninvasive bladder cancer: is it cost-effective in long-term application? A Germany-based cost analysis. Eur Urol 2007; 52: 142-7.
29. Hillemanns P, Reiff J, Stepp H, Soergel P. Lymph node metastasis detection of ovarian cancer by porphyrin fluorescence photodetection: case report. Lasers Med Sci 2007; 22: 131-5.
30. Bloznelyte-Plesniene L, Rutkovskiene L. Photodynamic therapy of malignant and benign tumours in Lithuania. Acta medica Lituanica 2007; 14 (3): 193-200.
31. Liutkeviciute-Navickiene J, Mordas A, Rutkovskiene L, Bloznelyte-Plesniene L. Skin and mucosal fluorescence diagnosis with different light sources. Eur J Dermatol 2009; 19 (2): 135-40.
32. Rigual NR, Thankappan K, Cooper M, Sullivan MA, Dougherty T, Popat SR, et al. Photodynamic therapy for head and neck dysplasia and cancer. Arch Otolaryngol Head Neck Surg 2009; 135 (8): 784-8.
33. Schweitzer VG, Somers ML. Photofrin-mediated photodynamic therapy for treatment of early stage (Tis-T2N0N0)SqCCa of oral cavity and oropharynx. Lasers Surg Med 2010; 42: 1-8.
34. Canavesi C, Fournier F, Cassarly WJ, Foster TH, Rolland JP. Illumination devices for photodynamic therapy of the oral cavity. Biomed Opt Express 2010; 1 (5): 1480-90.
35. Karakullukcu B, Oudenaarde K, Copper MP, Klop WMC, Veen R, Wildeman M, et al. Photodynamic therapy of early stage oral cavity and oropharynx neoplasms: an outcome analysis of 170 patients. Eur Arch Otorhinolaryngol 2011; 268: 281-8.
36. Meissner O, Ostermeier M, Hoheisel M, inventors. Combined OCT catheter device and method for combined optical coherence tomography (OCT) diagnosis and photodynamic therapy (PDT). United States patent US 20090240154. 2009 Sep 24.
37. Zenk W, Dietel W, Schleier P, Gunzel S. [Visualizing carcinomas of the mouth cavity by stimulating synthesis of fluorescent protoporphyrin IX]. Mund Kiefer Gesichtschir 1999; 3 (4): 205-9.
38. Lane PM, Gilhuly T, Whitehead P, Zeng H, et al. Simple device for the direct visualization of oral-cavity tissue fluorescence. J Biomed Opt 2006; 11: 024006 1-7.
39. Toursounidis T, Upile Ch, Betz S, Shah P, et al. Fluorescence spectroscopy in the detection of oral dysplasia. Head Neck Oncol 2009; 1 (Suppl 1): P3.
40. Heintzelman DL, Utzinger U, Fuchs H, et al. Optimal excitation wavelengths for in vivo detection of oral neoplasia using fluorescence spectroscopy. Photochem Photobiol 2000; 72 (1): 103-13.
41. Smith PW. Fluorescence emission-based detection and diagnosis of malignancy. J Cellul Biochem Suppl 2002; 39: 54-9.
42. Pathak N, Davis L, Hsiang YN, et al. Detection of squamous neoplasia by fluorescence imaging comparing porfimer sodium fluorescence to tissue autofluorescence in the hamster cheek-pouch model. Am J Surg 1995; 170: 423-6.
43. Westra WH, Sidransky D. Fluorescence visualization in oral neoplasia: shedding light on an old problem. Clin Cancer Res 2006; 12 (22): 6594-7.
44. Friesen SA, Hjortland GO, Madsen SJ, Hirschberg H, Engebraten O, Nesland JM, Peng Q. 5-aminolevulinic acid-based photodynamic detection and therapy of brain tumors. Int J Oncol 2002; 21: 577-82.
45. Tope WD, Ross EV, Kollias N, Martin A, et al. Protoporphyrin IX fluorescence induced in basal cell carcinoma by oral delta-aminolevulinic acid. Photochem Photobiol 1998; 67: 249-55.
46. Johansson J, Berg R, Svanberg K, Svanberg S. Laser-induced fluorescence studies of normal and malignant tumour tissue of rat following intravenous injection of delta-amino levulinic acid. Lasers Surg Med 1997; 20: 272-9.
47. Rick K, Sroka R, Stepp H, Kriegmair M, et al. Pharmacokinetics of 5-aminolevulinic acid-induced protoporphyrin IX in skin and blood. J Photochem Photobiol B 1997; 40: 313-9.
48. Leunig A, Rick K, Stepp H, Gutmann R, et al. Fluorscence imaging and spectroscopy of 5-aminolevulinic acid induced protoporphyrin IX for the detection of neoplastic lesions in the oral cavity. Am J Surg 1996; 172: 674-7.
49. Campbell DL, Gudgin-Dickson EF, Forkert PG, et al. Detection of early stages of carcinogenesis in adenomas of murine lung by 5-aminolevulinic acid-induced protoporphyrin IX fluorescence. Photochem Photobiol 1996; 64: 676-82.
50. Gronlund-Pakkanen S, Makinen K, Talja M, Kuusisto A, Alhava E. The importance of fluorescence distribution and kinetics of ALA-induced PpIX in the bladder in photodynamic therapy. J Photochem Photobiol B 1997; 438: 269-73.
51. Bloznelyte-Plesniene L, Mordas A, Liutkeviciu̅te-Navickiene J, Cepulis V, Valuckas KP, Ostapenko V, Rutkovskiene L, Venius J. Intraarterine fotodinamine diagnostika. Patentas (Nr. LT 5823 B), 2012-04-25.
52. Irion KM, Hahn R. Use of 5-aminolevulinic acid or a derivate there of for photodynamic diagnosis and/or photodynamic therapy. Patent (No.: US 6,860,879 B2), 2005-03-01.
53. Godie O. Device for detecting pericardial effusion. Patent (No.: US 6,351,667 B1), 2002-02-26.
54. Degen TW, Thommen DT, Johnson NL. Apparatus and methods for treating intracorporeal fluid accumulation. Patent (No.: US 2012/0209085 A1), 2012-08-16.
55. Hideki Sakurai et al: "Basic study on photodynamic therapy in liver cell carcinoma with reference to experimetal liver tumor in rabbits and distribution of photosensitive PH-1126", Journal of Tokyo Medical College, vol. 48, no. 6, 25 July 1990, pages 786-794.
56. Flvia Rodrigues de Oliveira Silva et al:" Enhancement of blood porphyrin emission intensity with aminolevulinic acid administration: A new concept for photodynamic diagnosis of early prostate cancer", Photodiagnosis and Photodynamic Therapy, vol. 8, no.1, 17 November 2011, pages 7-13.
57. Malini Olivo et al: "A new frontier in hypericin-mediated diagnosis of cancer with current optical technologies", Annals of biomedical engineering, Kluwer academic publishers-plenum publishers, NE, vol. 40, no.2, 29 November 2011, pages 460-473.
58. Pytel Akos et al:"New aspect of photodynamic diagnosis of bladder tumors:Fluerescence cytology", Urology, Belle Mead, NJ, US, vol. 59, no. 2, 1 February 2002, pages 216-219 [58].
59. Stephan Tauber et al: "Fluorescence cytology of the urinary bladder", Urology, vol. 61, no. 5, 1 May 2003, pages 1067-1071.
60. Stephan Tauber et al.: "Integral spectrophotometric analysis of 5-aminolaevulinic acid-induced fluorescence cytology of the urinary bladder", BJU international, vol. 97, no. 5, 1 May 2006, pages 992-996.
61. Muhammad W. Saif et al.: "Management of ascites due to gastrointestinal malignancy", Ann Saudi Med.2009 Sep-Oct; 29(5):, 1 March 2009 (2009-03-01), pages 369-377., XP055183892.
62. M. Fiegl: "Combination of Cytology, Fluorescence In Situ Hybridization for Aneuploidy, and Reverse-Transcriptase Polymerase Chain Reaction for Human Mammaglobin/Mammaglobin B Expression Improves Diagnosis of Malignant Effusions", Journal of Clinical Oncology, vol. 22, no. 3, 22 December 2003 (2003-12-22), pages 474-483, XP055183895, ISSN: 0732-183X, DOI: 10.1200/JCO.2004.06.063
63. Ben Davidson: "Biological Characteristics of Cancers Involving the Serosal Cavities - Critical Reviews(TM) in Oncogenesis, Volume 13, 2007, Issue 3 - Begell House Digital Library", , 1 January 2007 (2007-01-01), XP055183906.
64. Akihiko Kawahara et al.: "Cytological features of cystadenocarcinoma in cyst fluid of the parotid gland: Diagnostic pitfalls and literature review", Diagnostic Cytopathology, 1 January 2009 (2009-01-01), pages NA-NA, XP055183908, ISSN: 8755-1039, DOI: 10.1002/dc.21232.
65. Ter Borg E. J. et al: "Monoarthritis of the elbow due to metastatic colon carcinoma: diagnosis based on the presence of adenocarcinoma cells in synovial fluid", RHEUMATOLOGY INTERNATIONAL ; CLINICAL AND EXPERIMENTAL INVESTIGATIONS, SPRINGER, BERLIN, DE, vol. 28, no. 11, 26 April 2008 (2008-04-26), pages 1177-1178, XP019626043, ISSN: 1437-160X.
66. Anja C. Roden et al.: "Seroma-associated primary anaplastic large-cell lymphoma adjacent to breast implants: an indolent T-cell lymphoproliferative disorder", Modern Pathology, vol. 21, no. 4, 25 January 2008 (2008-01-25), pages 455-463, XP055183953, ISSN: 0893-3952, DOI: 10.1038/modpathol.3801024.
67. H. L. Gornik: "Abnormal Cytology Predicts Poor Prognosis in Cancer Patients With Pericardial Effusion", Journal of Clinical Oncology, vol. 23, no. 22, 6 June 2005 (2005-06-06), pages 5211-5216, XP055183957, ISSN: 0732-183X, DOI: 10.1200/JCO.2005.00.745.

## Claims

1. Method of photodynamic identification of pathological tissue fluids malignancy level in vitro comprising subjecting a pre-collected pathological tissue fluid sample, originating from a human subject that has been administered a photosensitizer prior to collecting the body fluid sample, to irradiation by light, observing said sample for fluorescence glow, **characterized in that** the photosensitizer is selected from photofrin or photohem or photosan, administered to a patient respectively in an amount of 2 mg/kg, 2.5 mg/kg, 2 mg/kg of body weight and **in that** said pre-collected pathological tissue fluid sample is irradiated by light in the period of 24-144 hours after administration of any of said photosensitizers whereby the sensitivity and specificity being 96% and 100% respectively remains such for 144 hours after administration of said photosensitizers.

2. Use of photodynamic identification method of claim 1 in differential photodynamic diagnostics of pathologies.

## Patentansprüche

1. Verfahren zur photodynamischen Identifizierung von pathologischem Gewebeflüssigkeits-Malignitätsniveau in vitro, umfassend das Vornehmen einer vorgesammelten pathologischen Gewebeflüssigkeitsprobe, die von einem menschlichen Subjekt stammt, dem ein Photosensibilisator vor dem Sammeln der Körperflüssigkeitsprobe verabreicht wurde, zur Bestrahlung durch Licht, das Beobachten der oben erwähnten Probe für Fluoreszenzglühen, das Verfahren wird **dadurch gekennzeichnet, dass** der Photosensibilisator aus Photofrin oder Photohem oder Photosan ausgewählt ist, der einem Patienten jeweils in einer Menge von 2 mg / kg, 2,5 mg / kg, 2 mg / kg Körpergewicht verabreicht wird, die oben erwähnte vorgesammelte pathologische Gewebeflüssigkeitsprobe wird in der Zeitspanne von 24-144 Stunden nach der Verabreichung irgendeines der Photosensibilisatoren durch Licht bestrahlt, wobei die Empfindlichkeit und Spezifität 96% beziehungsweise 100% für 144 Stunden nach der Verabreichung der oben erwähnten Photosensibilisatoren bestehen.

2. Verwendung des photodynamischen Identifikationsverfahrens nach Anspruch 1 bei der differentiellen photodynamischen Diagnostik von Pathologien.

## Revendications

1. Il s'agit ici d'une méthode d'identification photodynamique de malignité de fluides tissulaires pathologiques (in vitro) (pathologiques étant les fluides et non pas les tissus), la méthode comprenne a) la soumission d'un échantillon de fluide de tissu pathologique, provenant d'un patient (humain) auquel, avant de recueillir l'échantillon de fluide corporel, un photosensibilisateur a été introduit, et b) l'irradiation de l'échantillon susmentionné avec de la lumière nécessaire afin d'observer un rayonnement fluorescent, la méthode est **caractérisée par le fait que** le photosensibilisant choisi est la photofrine ou la photohem ou le photosan, prescrivant à un patient respectivement en une quantité de 2 mg / kg, de 2,5 mg / kg, de 2 mg / kg de poids corporel, la méthode est caractérisée aussi **par le fait que** l'échantillon de fluide tissulaire pathologique susmentionné est irradié avec une certaine lumière pendant la période de 24 à 144 heures après l'insertion d'un quelconque photosensibilisateur, la sensibilité et la spécificité de cette méthode est de 96% et 100% respectivement pendant 144 heures après l'insertion des photosensibilisateurs.

2. Utilisation de la méthode d'identification photodynamique selon la revendication 1 dans le diagnostic photodynamique différentiel de pathologies.
